# EUROPEAN PATENT SPECIFICATION

(11) **EP 4 279 004 B1**
(45) Date of publication and mention of the grant of the patent: **13.08.2025**
(21) Application number: 23196484.2
(22) Date of filing: 25.10.2018
(51) Int. Cl.: A61B 18/14, A61B 5/05, A61B 5/06, A61B 5/00, A61B 18/00, A61B 34/20

(54) **USE OF ELECTROMAGNETIC FIELDS IN IRE DEVICE DELIVERY AND THERAPY MONITORING**
VERWENDUNG VON ELEKTROMAGNETISCHEN FELDERN BEI DER ABGABE EINER IRE-VORRICHTUNG UND THERAPIEÜBERWACHUNG
UTILISATION DE CHAMPS ÉLECTROMAGNÉTIQUES DANS L'ADMINISTRATION DE DISPOSITIF IRE ET LA SURVEILLANCE DE THÉRAPIE

(30) Priority: 26.10.2017 US 201762577539 P
(43) Date of publication of application: 22.11.2023
(62) Divisional of application: 18800440.2
(73) Proprietor: Boston Scientific Scimed, Inc., Maple Grove, MN 55311 (US); Mayo Foundation for Medical Education and Research, Rochester, Minnesota 55905 (US)
(72) Inventor: ROHL, James P., Prescott Wisconsin 54021 (US); GRUBA, Sarah M., Vadnais Heights Minnesota 55127 (US); PAGORIA, Douglas D., Forest Lake Minnesota 55025 (US); HEIN, Matthew, Eden Prairie Minnesota 55346 (US); ASIRVATHAM, Samuel J., Rochester Minnesota 55906-8971 (US); WITT, Chance M., Rochester Minnesota 55901-2244 (US); KAPA, Suraj, Rochester Minnesota 55902 (US)
(74) Representative: Vossius & Partner Patentanwälte Rechtsanwälte mbB

(56) References cited:
- WO-A1-2016/181316
- WO-A2-2016/181317
- US-A1- 2004 097 803

## Description

### CROSS-REFERENCE TO RELATED APPLICATION

This application claims priority to Provisional Application No. 62/577,539, filed October 26, 2017.

### TECHNICAL FIELD

The present disclosure generally relates to systems and methods for providing a therapy to a patient. More particularly, the present disclosure relates to apparatuses, systems, and methods assessing electroporation.

### BACKGROUND

Electroporation may permeate cell membranes through exposure to certain electric pulses. Irreversible electroporation may be alternative for the ablation of undesired tissue. Electroporation without thermal effect to ablate tissue may avoid thermal damage to target tissue or other tissue surrounding the target tissue and/or ablates cells without damaging the blood vessel structure.

Atrial fibrillation is an irregular and often rapid heart rate that commonly causes poor blood flow to the body. Ablation procedures, including ablation of thoracic veins such as the pulmonary vein, may be a treatment for atrial fibrillation. During pulmonary vein ablation, for example, catheters are inserted into the atrium and energy is delivered to the tissue of the pulmonary vein and/or near the ostia of the pulmonary veins in the left atrium. Ablative energy, by electroporation, may be used in other areas of the heart, other veins, or blood vessels.

Analysis and/or tracking of the electroporated tissue may be useful in effectively and accurately ablating tissue using electroporation. Presently, it may take days or months to assess the irreversible nature of the procedure.

WO 2016/181317 A2 relates to systems and methods for planning of catheter ablation procedures, and in particular for planning of the placement of lesions and/or parameters used in ablation. In some embodiments, planning is based on thermal and/or dielectric simulation of lesions, individualized to the anatomy of the particular patient. Optionally, a plan comprises planning of a path along which an ablation lesion is to be formed, the ablation lesion optionally comprising one or more sub-lesions.

WO 2016/181316 A1 relates to a computerized method of tracking a position of an intra-body catheter, comprising: physically tracking coordinates of the position of a distal portion of a physical catheter within the physical body portion of the patient according to physically applied plurality of electrical fields within the body portion and measurements of the plurality of electrical fields performed by a plurality of physical electrodes at a distal portion of the physical catheter; registering the physically tracked coordinates with simulated coordinates generated according to a simulation of a simulated catheter within a simulation of the body of the patient, to identify differences between physically tracked location coordinates and the simulation coordinates; correcting the physically tracked location coordinates according to the registered simulation coordinates; and providing the corrected physically tracked location coordinates for presentation.

### SUMMARY

The invention is defined in the appended claims. The present disclosure provides also further examples useful for better understanding of the invention. In particular, the disclosed methods are not claimed and do not form part of the invention.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 shows an example electroporation device at a target tissue region within patient's heart in accordance with embodiments of the disclosure.
FIG. 2A shows an example electroporation device and electromagnetic field in accordance with embodiments of the disclosure.
FIG. 2B shows the electroporation device, as shown in FIG. 2A, at a target tissue region prior to application of electroporation energy in accordance with embodiments of the disclosure.
FIG. 2C shows the electroporation device, as shown in FIGs. 2A-B, at a target tissue region after application of electroporation energy in accordance with embodiments of the disclosure.
FIG. 3 shows a partial cross-sectional illustration of an example electroporation device in accordance with embodiments of the disclosure.
FIG. 4 shows an example electroporation system arranged at a target tissue region within patient's heart in accordance with embodiments of the disclosure.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

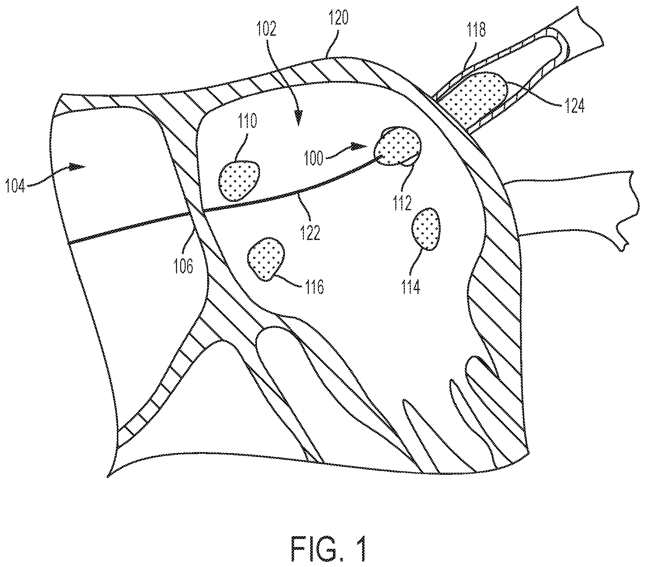

FIG. 1 shows an example electroporation device 100 at a target tissue region within patient's heart 120 in accordance with embodiments of the disclosure. The heart 120 shown in FIG. 1 may be undergoing a pulmonary vein ablation procedure using a electroporation device 100 in accordance with various aspects discussed herein. The electroporation device 100 may be used in other blood vessels or arteries or other portions of the heart such as the left atrial appendage. The electroporation device 100 may include a catheter having an elongate body 122 that is connected to a balloon structure 124. The electroporation device 100 may be connected to an ablation energy source and controller (e.g., radiofrequency (RF) or direct current (DC) system not shown) and one or more liquid sources (not shown), both of which are located external to the patient. The balloon structure 124 may be located near the distal end of elongate body 122. One or more interior chambers of the balloon structure 124 may be in fluid communication with a liquid delivery lumen arranged within the elongate body 122. The liquid delivery lumen is used to convey the one or more liquids from the source external to the patient into the balloon structure 124. The elongate body 122 and the balloon structure 124 may be delivered to a tissue region to which ablation energy may be applied.

As shown in FIG. 1, the elongate body 122 may be positioned in the left atrium 102 of the patient's heart 120. More specifically and in certain instances, the electroporation device 100 may enter the right atrium 104 of heart 120 through a femoral vein and the inferior vena cava (not shown). The electroporation device 100 may be passed through a puncture in an atrial septum 106 to access left atrium 102. From the left atrium 102, the balloon catheter electroporation device 100 may be positioned through any of the pulmonary vein ostia 110, 112, 114, or 116 to enter a pulmonary vein such as pulmonary vein 118. In certain instances, the electroporation device 100 may be an over-the-wire device that is delivered over or on a pre-placed guidewire or a delivery catheter/sheath and/or be self steerable or rapid exchange catheter may be used to assist in the insertion and placement of the electroporation device 100.

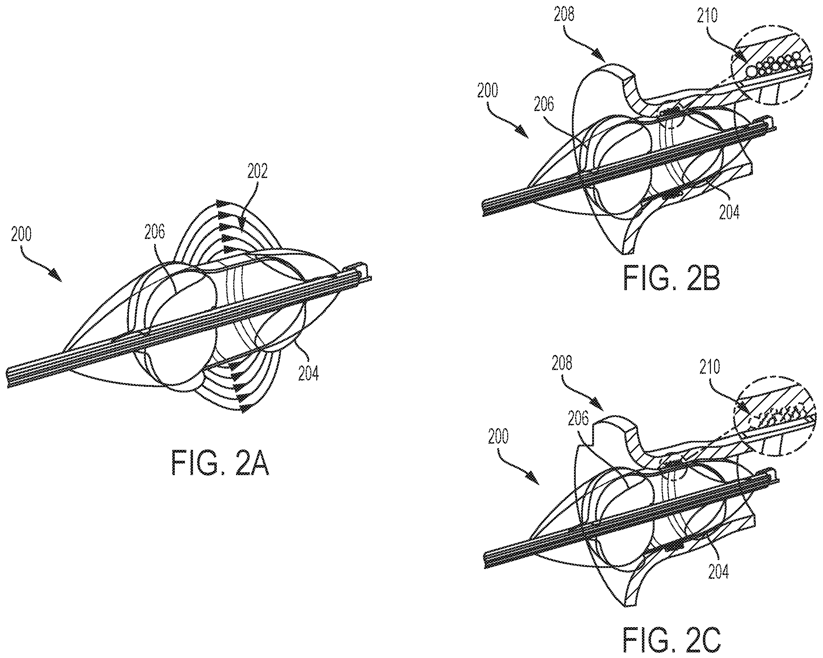

After positioning of the electroporation device 100 at the tissue region (within the pulmonary vein 118 as shown in FIG. 2), the balloon structure 124 may be expanded. The balloon structure 124 may be inflated using a liquid (e.g., saline, a pharmacological agent, or a combination thereof) as the inflation medium. In instances where the balloon structure 124 is positioned within a vessel such as the pulmonary vein 118, the inflation of balloon structure 124 may cause the outer surface of balloon structure 124 to contact an inner wall of vessel such as the pulmonary vein 118. In certain instances, ablation energy may be applied through one or more electrodes (not shown) arranged within the balloon structure 124 to initiate the modulation of target neural fibers. In addition, one or more portions of the balloon structure 124 may have a permeability such that a liquid may exude, elute, weep, or otherwise be transmitted from therethrough. In certain instances, the liquid may be an anti-stenotic pharmaceutical agent that may contact the inner wall of pulmonary vein 118.

The ablation energy may be applied through one or more portions of the balloon structure 124 by an electric field generated by the external source/controller and transferred through wires within one or more lumens of the elongate body 122 to electrodes (not shown) arranged with the balloon structure 124. The electric energy can be transmitted to the inner wall of pulmonary vein 118 directly from the electrodes on the surface of balloon structure 124 or from the electrodes within the balloon structure 124. The electric field may at least partially cause apoptotic cell death and/or non-thermal necrosis to the tissue receiving the ablation energy.

In certain instances, the electric field may be generated by applying direct current to the one or more electrodes arranged within the balloon structure 124. In addition, the use of direct current may cause apoptotic cell death and/or non-thermal necrosis to the tissue receiving the ablation energy. The direct current may form pores in the cells of the wall of the pulmonary vein 118 that are irreversible (e.g., the pores do not close). The balloon structure 124 being in contact with the wall of the pulmonary vein 118 may provide controlled and direct ablation of a target area while mitigating against down-stream proliferation of the ablation energy.

FIG. 2A shows an example electroporation device 200 and electromagnetic field 202 in accordance with embodiments of the disclosure. electroporation device 200 includes a distal electrode 204 and a proximal electrode 206. Current may be applied to the distal electrode 204 and the proximal electrode 206 to create the electromagnetic field 202 between the distal electrode 204 and the proximal electrode 206. In certain instances, direct current may be pulsed from one of the distal electrode 204 and the proximal electrode 206 to the other of the distal electrode 204 and the proximal electrode 206 to create the electromagnetic field 202. The distal electrode 204 and the proximal electrode 206 may be opposite charged to create the electromagnetic field 202. In certain instances, the distal electrode 204 and the proximal electrode 206 are electrically separated or isolated in order to oppositely charge the distal electrode 204 and the proximal electrode 206 to create the electromagnetic field 202.

The electromagnetic field 202 may utilized to verify that the electroporation therapy applied by the electroporation device 200 is delivered at a desired location and/or at a desired effectiveness. Tissue affected by the electromagnetic field 202 may change (e.g., in density) as compared to unaffected tissue.

FIG. 2B shows the electroporation device 200, as shown in FIG. 2A, at a target tissue region 208 prior to application of electroporation energy in accordance with embodiments of the disclosure. As shown in FIG. 2B, a group of cells 210 are highlighted in the target tissue region 208. The group of cells 210 have not been affected (e.g., voided or a ions have not evacuated the group of cells 210) as energy has not been applied to the electroporation device 200 at this point.

FIG. 2C shows the electroporation device 200, as shown in FIGs. 2A-B, at a target tissue region 208 after application of electroporation energy in accordance with embodiments of the disclosure. The same group of cells 210 shown in FIG. 2B are shown as affected as a result of the energy applied to the electroporation device 200. The group of cells 210 may swell or void after electroporation which would change the density of the group of cells 210. The electromagnetic field 202, shown in FIG. 2A, voids or swells the group of cells 210. In comparing the group of cells 210 in FIG. 2B (unaffected cells) and the group of cells 210 in FIG. 2C (affected cells), a difference in density is present therebetween. Local magnetic field variations, or magnetic field gradients, may introduced due to the "voiding" or "swelling" of the impacted cells 210 (shown in FIG. 2C). The localized gradients can be utilized to define a successful application of electroporation energy by the electroporation device 200 within the target tissue region 208.

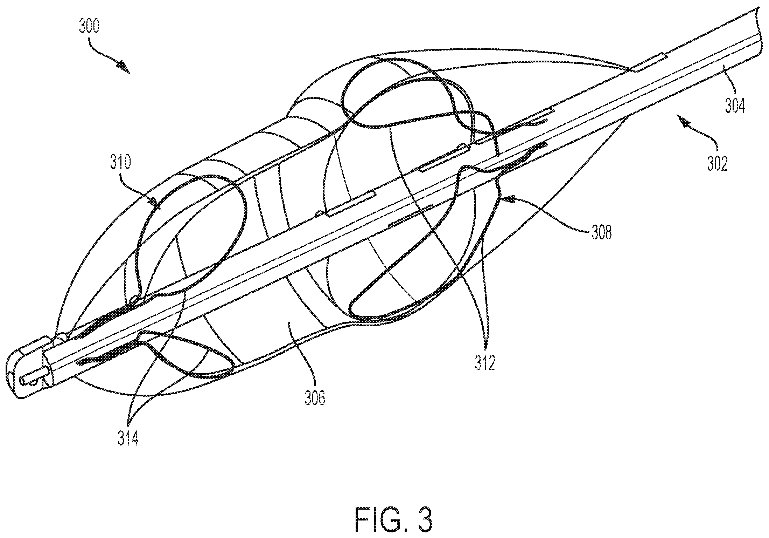

FIG. 3 shows a partial cross-sectional illustration of an example electroporation device 300 in accordance with embodiments of the disclosure. The electroporation device 300 may include a catheter 302 sized and shaped for vascular access that has an elongate body 304 extending between a proximal end and a distal end of the catheter 302. A distal portion of the catheter 302 and the elongate body 304 is shown in FIG. 3. The electroporation device 300 may also include a balloon structure 306 arranged near the distal end of the elongate body 304. The balloon structure 306 may be configured to inflate in response to a liquid or inflation medium being provided thereto.

The electroporation device 300 may also include one or more electrodes arranged on or within the balloon structure 306. As shown in FIG. 3, the apparatus includes a proximal electrode 308 arranged near a proximal end and on or within the balloon structure 306 and a distal electrode 310 arranged near a distal end and on or within the balloon structure 306. The proximal electrode 308 and the distal electrode 310 may be configured to deliver energy to a tissue region. In certain instances, the proximal electrode 308 and the distal electrode 310 may be configured to delivery energy in response to a direct current applied thereto. As a result of the direct current applied to the proximal electrode 308 and the distal electrode 310, an electromagnetic field develops to delivery electroporation energy to the tissue region. The direct current may be applied in pulse bursts applied that results in electroporation energy delivered by the electroporation device 300.

As shown in FIG. 3, the proximal electrode 308 includes portions 312 that extend toward the distal electrode 310 and the distal electrode 310 includes portions 314 that extend toward the proximal electrode 308. These portions 312, 314 may control a shape of the electromagnetic field that develops as a result of the pulse bursts applied to the proximal electrode 308 and the distal electrode 310. In addition, the proximal electrode 308 are spaced apart by between 0 mm and 25 mm.

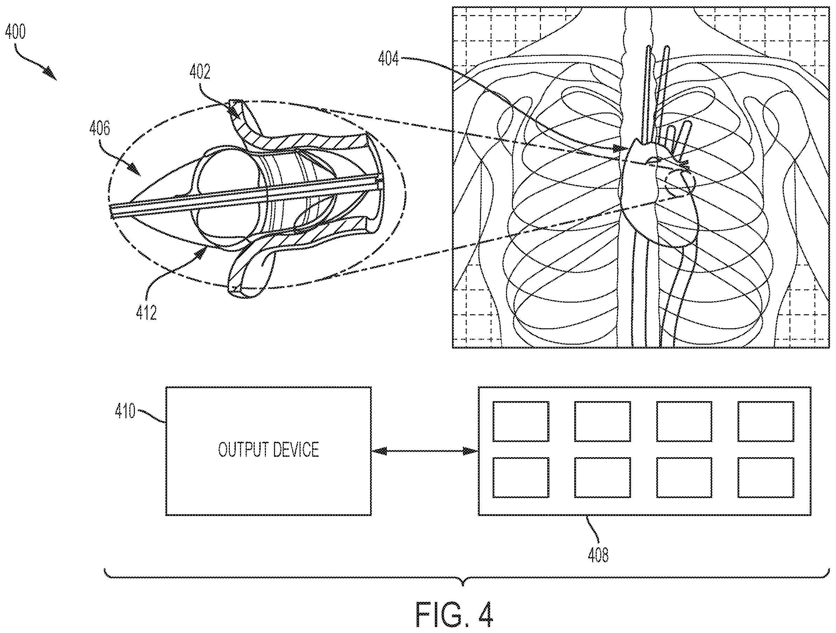

The balloon structure 306 may anchor the electroporation device 300 at a target location within a patient. In certain instances, the target location may be a vein or artery in a patient such as the pulmonary vein. Blood moving through the target location and/or pulsing of the patient's heart may affect movement of the electroporation device 300 and hinder the ability of the electroporation device 300 to accurate delivery electroporation energy. The electroporation device 300 may be used in connection with an array of sensors and an output device as shown in FIG. 4 and discussed in further detail below. The array of sensors and the output device may be configured to verify location of the electroporation device 300 and also may be configured to verify effectiveness of the electroporation energy applied by the electroporation device 300.

FIG. 4 shows an electroporation system 400 at a target tissue region 402 within patient's heart 404 in accordance with embodiments of the disclosure. The electroporation system 400 includes an electroporation device 406, an array of sensors 408, and an output device 410.

The array of sensors 408 are configured to sense an application of electroporation energy by the electroporation device 406 to determine a location of the electroporation device 406 within the patient. In certain instances, the array of sensors 408 are configured to sense an electromagnetic field generated by the electroporation device 406 (e.g., as discussed above with reference to FIGs. 2A-C) resulting from pulse bursts applied by the electroporation device 406. The array of sensors 408 may be a two-dimensional or three-dimensional array of magnoresistive sensors. The array of sensors 408 may be arranged alone one or more sides of the patient. In certain instances, the array of sensors 408 may be arranged beneath a patients back during the electroporation procedure. In addition, the array of sensors 408, when three-dimensional, may extend up at least a portion of the patient's side or sides.

The array of sensors 408 may be magnoresistive sensors such as Magneto-Resistance (AMR) sensors, Giant Magneto-Resistance (GMR) sensors, Magnetic Tunneling Junction (MTJ) sensors, Tunnel Magneto-Resistance (TMR) sensors, inductive sensors, fluxgate sensors, GMI (giant magnetoimpedance) sensors, hall sensors or other similarly configured sensors. In addition, the array of sensors 408 may be configured to measure an output magnetic field density emitted by the electroporation device 406 over a grid of the array of sensors 408.

The array of sensors 408 communicates, wirelessly or via a direct connection, with the output device 410. The output device 410 includes circuitry configured to measure the electromagnetic field generated by the electroporation device 406 and sensed by the array of sensors 408. In certain instances, the output device 410 is configured to measure a difference between in the electromagnetic field generated by the electroporation device 406 prior to application of pulse bursts and the electromagnetic field generated by the electroporation device 406 after to application of the pulse bursts. The difference measured by the output device 410 measures affected cells to determine the location of the electroporation device 406 within the patient. More specifically, the output device 410 is configured to measure the difference based on a change in cell density at the target tissue region 402.

Electroporated cells have a different density that cells that have not been electroporated, and the output device 410 may determine the difference in the electric fields, sensed by the array of sensors 408, of the affected and unaffected cells. Thus, the output device 410 may also be configured to verify effectiveness of the application of electroporation by the electroporation device 406 at the tissue region 402. In certain instances, the output device 410 is configured to measure the change in cell density to locate impacted and non-impacted cells in the tissue region. In addition, the output device 410 may be configured to display an indication of the impacted and non-impacted cells during application of the application of electroporation energy. In this manner, the output device 410 may provide a real-time display of the effectiveness of the electroporation device 406 during application of electroporation by the electroporation device 406 at the tissue region 402.

The electroporation device 406 may also include a balloon structure 412 configured to anchor the electroporation device 406 at the tissue region 402 within the patient. Blood moving through the tissue region and/or pulsing of the patient's heart 404 may affect movement of the electroporation device 406 and hinder the ability of the electroporation device 406 to accurate delivery electroporation energy. The balloon structure 412 may also facilitate application of the electroporation energy to the tissue region 402 by providing a contact area between the electroporation device 406 and the tissue region 402.

In addition, the array of sensors 408 and the output device 410 may provide real-time feedback to an operating physician using the electroporation device 406. The ability of the output device 410 to indicate location of the electroporation device 406 and effectiveness of the electroporation device 406 during use of the electroporation device 406 increases the effectives of the electroporation device 406.

Various modifications and additions which fall under the scope of the claims. can be made to the exemplary embodiments discussed without departing from the scope of the present invention.

## Claims

1. An apparatus for assessing electroporation therapy applied to a tissue region (208, 402) of a patient, the apparatus comprising:
an electroporation device (100, 200, 300, 406) comprising:
a catheter (302) sized and shaped for vascular access and including an elongate body (122, 304) extending between a proximal end and a distal end,
a balloon structure (124, 306) arranged near the distal end of the elongate body, and
one or more electrodes (204, 206, 308, 310) arranged on or within the balloon structure and configured to deliver electroporation energy to the tissue region; and
an array of sensors (408) configured to be positioned on the patient and to sense an application of the electroporation energy by the electroporation device to determine a location of the electroporation device within the patient.

2. The apparatus of claim 1, wherein the array of sensors (408) are at least one of a two dimensional array of magnetoresistive sensors and three dimensional array of magnetoresistive sensors.

3. The apparatus of claim 2, wherein the magnetoresistive sensors include at least one of Magneto-Resistance (AMR) sensors, Giant Magneto-Resistance (GMR) sensors, Magnetic Tunneling Junction (MTJ) sensors, and Tunnel Magneto-Resistance(TMR) sensors, inductive sensors, fluxgate sensors, GMI (giant magnetoimpedance) sensors, and hall sensors.

4. The apparatus of any one of claims 1 to 3, wherein the array of sensors (408) are configured to measure an output magnetic field density emitted by the electroporation device (100, 200, 300, 406) over a grid of the array of sensors.

5. The apparatus of any one of claims 1 to 4, wherein the one or more electrodes includes a proximal electrode (206, 308) arranged near a proximal end of the balloon structure (124, 306) and a distal electrode (204, 310) arranged near a distal end of the balloon structure, and the proximal electrode includes portions (312) extending toward the distal electrode and the distal electrode includes portions (314) extending toward the proximal electrode.

6. The apparatus of claim 5, wherein the proximal electrode (206, 308) and the distal electrode (204, 310) are spaced apart by between 0 mm and 25 mm.

7. The apparatus of any one of claims 1 to 6, further comprising an output device (410) coupled to the array of sensor (408), wherein the output device is further configured to verify effectiveness of the application of electroporation energy by the electroporation device (100, 200, 300, 406) at the tissue region.

8. An electroporation system (400), comprising:
the apparatus of any one of claims 1 to 6;
an output device (410) coupled to the array of sensors (408), wherein the output device is wirelessly or directly coupled to the array of sensors.

9. The electroporation system of claim 8, wherein the output device (410) is configured to measure a difference between the electromagnetic field generated by the electroporation device (100, 200, 300, 406) prior to application of pulse bursts and the electromagnetic field generated by the electroporation device after application of the pulse bursts.

10. The electroporation system of claim 9, wherein the output device (410) is configured to determine the location of the electroporation device (100, 200, 300, 406) within the patient based on the measured difference.

11. The electroporation system of any of the preceding claims, wherein the one or more electrodes (204, 206, 308, 310) are arranged within the balloon structure.

## Patentansprüche

1. Vorrichtung zur Festlegung einer Elektroporationstherapie, die auf einen Gewebebereich (208, 402) eines Patienten angewendet wird, wobei die Vorrichtung aufweist:
eine Elektroporationsvorrichtung (100, 200, 300, 406), die aufweist:
einen Katheter (302), der für einen vaskularen Zugang dimensioniert und geformt ist und einen länglichen Körper (122, 304) aufweist, der sich zwischen einem proximalen Ende und einem distalen Ende erstreckt;
eine Ballonstruktur (124, 306), die in der Nähe des distalen Endes des länglichen Körpers eingerichtet ist, und
eine oder mehrere Elektroden (204, 206, 308, 310), die auf oder innerhalb der Ballonstruktur eingerichtet und konfiguriert sind, um Elektroporationsenergie an den Gewebebereich zu liefern; und
eine Anordnung von Sensoren (408), die konfiguriert ist, um auf dem Patienten positioniert zu werden und eine Anwendung der Elektroporationsenergie durch die Elektroporationsvorrichtung abzutasten, um einen Ort der Elektroporationsvorrichtung innerhalb des Patienten zu bestimmen.

2. Vorrichtung nach Anspruch 1, wobei die Anordnung von Sensoren (408) eine zweidimensionale Anordnung magnetoresistiver Sensoren und/oder eine dreidimensionale Anordnung magnetoresistiver Sensoren ist.

3. Vorrichtung nach Anspruch 2, wobei die magnetoresistiven Sensoren magnetoresistive (AMR-) Sensoren und/oder magnetoresistive Giant- (GMR-) Sensoren und/oder magnetische Tunnelübergangs- (MTJ-) Sensoren und/oder magnetoresistive Tunnel- (TMR-) Sensoren und/oder induktive Sensoren und/oder Luftspaltsensoren und/oder GMI (Magnetoimpedanz-Giant-) Sensoren und/oder Hall-Sensoren aufweisen.

4. Vorrichtung nach einem der Ansprüche 1 bis 3, wobei die Anordnung von Sensoren (408) konfiguriert ist, um eine von der Elektroporationsvorrichtung (100, 200, 300, 406) emittierte Ausgangsmagnetfelddichte über ein Gitter der Sensorenanordnung zu messen.

5. Vorrichtung nach einem der Ansprüche 1 bis 4, wobei die eine oder mehreren Elektroden eine proximale Elektrode (206, 308), die in der Nähe eines proximalen Endes der Ballonstruktur (124, 306) eingerichtet ist, und eine distale Elektrode (204, 310), die in der Nähe eines distalen Endes der Ballonstruktur eingerichtet ist, aufweist, und wobei die proximale Elektrode Abschnitte (312), die sich in Richtung der distalen Elektrode erstrecken, aufweist und die distale Elektrode Abschnitte (314), die sich in Richtung der proximalen Elektrode erstrecken, aufweist.

6. Vorrichtung nach Anspruch 5, wobei die proximale Elektrode (206, 308) und die distale Elektrode (204, 310) zwischen 0 mm und 25 mm voneinander beabstandet sind.

7. Vorrichtung nach einem der Ansprüche 1 bis 6, die ferner eine Ausgabevorrichtung (410) aufweist, die mit der Sensorenanordnung (408) gekoppelt ist, wobei die Ausgabevorrichtung ferner konfiguriert ist, um die Wirksamkeit der Anwendung von Elektroporationsenergie durch die Elektroporationsvorrichtung (100, 200, 300, 406) an dem Gewebebereich zu überprüfen.

8. Elektroporationssystem (400), das aufweist:
die Vorrichtung nach einem der Ansprüche 1 bis 6;
eine Ausgabevorrichtung (410), die mit der Sensorenanordnung (408) gekoppelt ist, wobei die Ausgabevorrichtung (410) drahtlos oder direkt mit der Sensorenanordnung gekoppelt ist.

9. Elektroporationssystem nach Anspruch 8, wobei die Ausgabevorrichtung (410) dazu konfiguriert ist, eine Differenz zwischen dem von der Elektroporationsvorrichtung (100, 200, 300, 406) erzeugten elektromagnetischen Feld vor einer Anwendung von Impulsbursts und dem von der Elektroporationsvorrichtung erzeugten elektromagnetischen Feld nach der Anwendung der Impulsbursts zu messen.

10. Elektroporationssystem nach Anspruch 9, wobei Ausgabevorrichtung (410) dazu konfiguriert ist, den Ort der Elektroporationsvorrichtung (100, 200, 300, 406) innerhalb des Patienten basierend auf der gemessenen Differenz zu bestimmen.

11. Elektroporationssystem nach einem der vorhergehenden Ansprüche, wobei die eine oder mehreren Elektroden (204, 206, 308, 310) innerhalb der Ballonstruktur eingerichtet ist/sind.

## Revendications

1. Appareil pour déterminer un traitement par électroporation appliqué à une région tissulaire (208, 402) d'un patient, le dispositif comprenant :
un dispositif d'électroporation (100, 200, 300, 406) comprenant :
un cathéter (302) dimensionné et façonné pour un accès vasculaire et comprenant un corps allongé (122, 304) s'étendant entre une extrémité proximale et une extrémité distale,
une structure de ballonnet (124, 306) disposée près de l'extrémité distale du corps allongé, et
une ou plusieurs électrodes (204, 206, 308, 310) disposées sur ou à l'intérieur de la structure de ballonnet et configurées pour délivrer une énergie d'électroporation à la région tissulaire ; et
un réseau de capteurs (408) configurés pour être positionnés sur le patient et pour détecter une application de l'énergie d'électroporation par le dispositif d'électroporation pour déterminer l'emplacement du dispositif d'électroporation à l'intérieur du patient.

2. Appareil selon la revendication 1, dans lequel le réseau de capteurs (408) est au moins l'un parmi un réseau bidimensionnel de capteurs magnétorésistifs et un réseau tridimensionnel de capteurs magnétorésistifs.

3. Appareil selon la revendication 2, dans lequel les capteurs magnétorésistifs comprennent au moins l'un parmi les capteurs à magnétorésistance (AMR), les capteurs à magnétorésistance géante (GMR), les capteurs à jonction tunnel magnétique (MTJ), et les capteurs à magnétorésistance tunnel (TMR), les capteurs inductifs, les capteurs discriminateurs de flux, les capteurs GMI (magnéto-impédance géante), et les capteurs à effet Hall.

4. Appareil selon l'une quelconque des revendications 1 à 3, dans lequel le réseau de capteurs (408) est configuré pour mesurer une densité de champ magnétique de sortie émise par le dispositif d'électroporation (100, 200, 300, 406) sur une grille du réseau de capteurs.

5. Appareil selon l'une quelconque des revendications 1 à 4, dans lequel les une ou plusieurs électrodes comprennent une électrode proximale (206, 308) disposée près d'une extrémité proximale de la structure de ballonnet (124, 306) et une électrode distale (204, 310) disposée près d'une extrémité distale de la structure de ballonnet, et l'électrode proximale comprend des portions (312) s'étendant vers l'électrode distale et l'électrode distale comprend des portions (314) s'étendant vers l'électrode proximale.

6. Appareil selon la revendication 5, dans lequel l'électrode proximale (206, 308) et l'électrode distale (204, 310) sont espacées l'une de l'autre par entre 0 mm et 25 mm.

7. Appareil selon l'une quelconque des revendications 1 à 6, comprenant en outre un dispositif de sortie (410) couplé au réseau de capteurs (408), dans lequel le dispositif de sortie est en outre configuré pour vérifier l'efficacité de l'application d'énergie d'électroporation par le dispositif d'électroporation (100, 200, 300, 406) au niveau de la région tissulaire.

8. Système d'électroporation (400), comprenant :
l'appareil de l'une quelconque des revendications 1 à 6 ;
un dispositif de sortie (410), couplé au réseau de capteurs (408), lequel dispositif de sortie est couplé sans fil ou directement au réseau de capteurs.

9. Système d'électroporation selon la revendication 8, dans lequel le dispositif de sortie (410) est configuré pour mesurer la différence entre le champ électromagnétique généré par le dispositif d'électroporation (100, 200, 300, 406) avant l'application de salves d'impulsions et le champ électromagnétique généré par le dispositif d'électroporation après l'application des salves d'impulsions.

10. Système d'électroporation selon la revendication 9, dans lequel le dispositif de sortie (410) est configuré pour déterminer l'emplacement du dispositif d'électroporation (100, 200, 300, 406) à l'intérieur du patient sur la base de la différence mesurée.

11. Système d'électroporation selon l'une quelconque des revendications précédentes, dans lequel les une ou plusieurs électrodes (204, 206, 308, 310) sont disposées à l'intérieur de la structure de ballonnet.
